# EUROPÄISCHE PATENTSCHRIFT

(11) **EP 1 328 244 B1**
(45) Veröffentlichungstag und Bekanntmachung des Hinweises auf die Patenterteilung: **11.10.2006**
(21) Anmeldenummer: 01960429.7
(22) Anmeldetag: 29.06.2001
(51) Int. Cl.: A61K 8/49, A61Q 5/10

(54) **MITTEL UND VERFAHREN ZUM FÄRBEN VON KERATINFASERN**
AGENT AND METHOD FOR COLOURING KERATIN FIBRES
AGENT ET PROCEDE POUR COLORER DES FIBRES KERATINIQUES

(30) Priorität: 15.09.2000 DE 10045600
(43) Veröffentlichungstag der Anmeldung: 23.07.2003
(73) Patentinhaber: Wella Aktiengesellschaft, 64274 Darmstadt (DE)
(72) Erfinder: PASQUIER, Cécile, CH-1723 Marly (CH); CHARRIERE, Véronique, CH-1638 Morlon (CH); BRAUN, Hans-Jürgen, CH-3182 Überstorf (CH)
(86) Internationale Anmeldenummer: PCT/EP2001/007494
(87) Internationale Veröffentlichungsnummer: WO 2002/022093

(56) Entgegenhaltungen:
- WO-A-01/10379
- WO-A-01/47485
- US-A- 3 577 427
- US-A- 4 620 850
- US-A- 5 055 110

## Beschreibung

Gegenstand der vorliegenden Erfindung ist ein Mittel zur Färbung von Keratinfasern, wie zum Beispiel Seide, Wolle oder Haaren und insbesondere menschlichen Haaren, welches eine Kombination aus (i) mindestens einem 4-Nitro-2,1,3-benzoxadiazol-Derivat, einem 4-Nitro-2,1,3-benzothiadiazol-Derivat oder einem 4-Nitro-2,1,3-benzoselenadiazol-Derivat und (ii) mindestens einem Amin, Aminonitrobenzol oder Phenol enthält, sowie ein Verfahren zum Färben von Keratinfasern unter Verwendung dieses Färbemittels.

Haarfärbemittel werden je nach zu färbender Ausgangshaarfarbe und gewünschtem Endresultat hauptsächlich in den Bereich der Oxidationsfärbemittel oder der Tönungen unterteilt. Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen. Direktziehende Farbstoffe, insbesondere Nitrofarbstoffe werden ebenfalls oft in oxidativen Färbemitteln zur Erzeugung bestimmter Nuancen beziehungsweise zur Intensivierung der Farbe eingesetzt.

Es besteht jedoch weiterhin ein großer Bedarf für Färbemittel, die unter milden Bedingungen sowohl intensive als auch schonende Färbungen ermöglichen.

Überraschenderweise wurde nunmehr gefunden, dass durch die Verwendung einer Kombination aus mindestens einem 4-Nitro-2,1,3-benzoxadiazol-Derivat, einem 4-Nitro-2,1,3-benzothiadiazol-Derivat oder einem 4-Nitro-2,1,3-benzoselenadiazol-Derivat und (ii) mindestens einem Amin, Aminonitro-benzol oder Phenol, auf schonende Weise unter milden Bedingungen intensive Färbungen mit einer großen Nuancenvielfalt ermöglicht werden.

Gegenstand der vorliegenden Erfindung ist daher ein Mittel zur Färbung von Fasern (A), wie zum Beispiel Wolle, Seide, Baumwolle oder Haaren und insbesondere menschlichen Haaren, welches durch Vermischen zweier Komponenten (A1) und (A2) hergestellt wird und dadurch gekennzeichnet ist, dass die Komponente (A1) mindestens eine 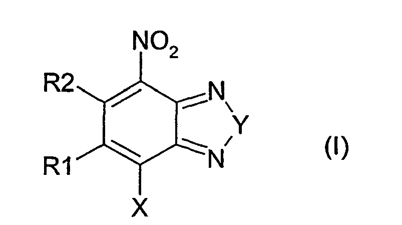 Verbindung der Formel (I) enthält,
worin X gleich einem Halogenatom (F, Cl, Br, J), einer Methoxy-Gruppe oder einer Ethoxy-Gruppe ist; Y gleich einem Sauerstoffatom, einem Schwefelatom oder einem Selenatom ist; und R1 und R2 gleich oder verschieden sein können und unabhängig voneinander Wasserstoff, ein Halogenatom (F, Cl, Br, J), eine (C₁-C₄)-Alkylgruppe, eine mit einem Halogenatom (F, Cl, Br, J) substituierte (C₁-C₄)-Alkylgruppe, eine (C₁-C₄)-Alkoxygruppe, eine Nitrogruppe, eine Acetamidogruppe oder eine NR^{a}R^{b}-Gruppe, wobei die Reste R^{a} und R^{b} gleich oder verschieden sein können und unabhängig voneinander Wasserstoff, eine (C₁-C₄)-Alkylgruppe, einen gegebenfalls substituierten aromatischen Carbozyklus oder eine (C₁-C₄)-Alkancarbonylgruppe darstellen, oder R^{a} und R^{b} gemeinsam mit dem Stickstoffatom einen heterozyklischen (C₃-C₆)- Rest (beispielsweise eine lmidazolidino-, Piperidino-, Pyrrolidino-, Pyrazolidino-, Piperazino- oder Morpholino-Gruppe) bilden;
und die Komponente (A2) mindestens eine Verbindung aus der Gruppe bestehend aus Aminen, Aminonitrobenzolen und Phenolen enthält.

Unter den Verbindungen der Formel (I) sind insbesondere die folgenden 4-Nitro-2,1,3-benzoxadiazole, 4-Nitro-2,1,3-benzothiadiazole und 4-Nitro-2,1,3-benzoselenadiazole zu nennen: 4-Chloro-7-nitro-2,1,3-benzoxadiazol; 4-Bromo-7-nitro-2,1,3-benzoxadiazol; 4-Fluoro-7-nitro-2,1,3-benzoxadiazol; 4-Methoxy-7-nitro-2,1,3-benzoxadiazol; 4-Ethoxy-7-nitro-2,1,3-benzoxadiazol; 4-Chloro-5,7-dinitro-2,1,3-benzoxadiazol; 5,7-Dichloro-4-nitro-2,1,3-benzoxadiazol; 5,7-Dibromo-4-nitro-2,1,3-benzoxadiazol; 7-Chloro-4-nitro-5-diethylamino-2,1,3-benzoxadiazol; 5-Amino-7-chloro-4-nitro-2,1,3-benzoxadiazol; 7-Chloro-4-nitro-5-(1-piperidinyl)-2,1,3-benzoxadiazol; 4-Chloro-7-nitro- 2,1,3-benzothiadiazol; 4-Bromo-7-nitro-2,1,3-benzothiadiazol; 4-Methoxy-7-nitro-2,1,3-benzothiadiazol; 4-Ethoxy-7-nitro-2,1,3-benzoxathiazol; 4-Chloro-5,7-dinitro-2,1,3-benzothiadiazol; 4-Bromo-5,7-dinitro-2,1,3-benzothiadiazol; 4,5-Dichloro-7-nitro-2,1,3-benzothiadiazol; 5,7-Dichloro-4-nitro-2,1,3-benzothiadiazol; 4,5-Dibromo-7-nitro-2,1,3-benzothiadiazol; 5,7-Dibromo-4-nitro-2,1,3-benzothiadiazol; 4,6-Dichloro-5,7-dinitro-2,1,3-benzothiadiazol; 4,6-Dibromo-5,7-dinitro-2,1,3-benzothiadiazol; 5-Chloro-7-fluoro-4-nitro-2,1,3-benzothiadiazol; 5-Anilino-7-bromo-4-nitro-2,1,3-benzothiadiazol; 5-Amino-7-chloro-4-nitro2,1,3-benzo-thiadiazol; 7-Bromo-5-(diethylamino)-4-nitro-2,1,3-benzothiadiazol; 4-Chloro-5-methyl-7-nitro-2,1,3-benzothiadiazol; 4-Bromo-5-methyl-4-nitro-2,1,3-benzothiadiazol; 7-Bromo-5-(bromomethyl)-7-nitro-2,1,3-benzothiadiazol; N-(7-Chloro-4-nitro-2,1,3-benzothiadiazol-5-yl)-acetamid; 4-Chloro-7-nitro-2,1,3-benzoselenadiazol; 4-Bromo-7-nitro-2,1,3-benzoselenadiazol; 4-Methoxy-7-nitro-2,1,3-benzoselenadiazol; 4-Ethoxy-7-nitro-2,1,3-benzoselenadiazol; 5,7-Dibromo-4-nitro-2,1,3-benzoselenadiazol; 5,7-Dichloro-4-nitro-2,1,3-benzoselenadiazol; 7-Bromo-5-methyl-4-nitro-2,1,3-benzoselenadiazol; 7-Bromo-5-(bromomethyl)-4-nitro-2,1,3-benzo-selenadiazol;
wobei die folgenden Verbindungen besonders bevorzugt sind: 4-Chloro-7-nitro-2,1,3-benzoxadiazol; 4-Bromo-7-nitro-2,1,3-benzoxadiazol; 4-Chloro-7-nitro-2,1,3-benzothiadiazol; 4-Bromo-7-nitro-2,1,3-benzothiadiazol; 4-Chloro-7-nitro-2,1,3-benzoselenadiazol und 4-Bromo-7-nitro-2,1,3-benzoselenadiazol.

Die Verbindungen der Formel (I) sind zum Teil im Handel erhältlich. Die Verbindungen der Formel (I) können jedoch auch nach aus der Literatur bekannten Syntheseverfahren, beispielsweise in Analogie zu den in Journal of Organic Chemistry 36 (1), (1971), Seiten 207-209; in Recueil Trav. Chim. Pays-Bas, 86 (12), Seiten 1159-1181 (1967); in Journal of Medicinal Chemistry, Vol.17 (2), Seiten 203-206 (1974); in Journal of the Chemical Society (B), (1967), Seiten 909-911, oder in der US-PS 3 577 427 beschriebenen Verfahren hergestellt werden.

Als Amine kommen aliphatische oder aromatische (isozyklische oder heterozyklische) Verbindungen mit mindestens einer Aminogruppe in Betracht, während als Aminonitrobenzole aromatische (isozyklische oder heterozyklische) Verbindungen mit mindestens einer Nitrogruppe und mindestens einer Aminogruppe in Betracht kommen, und als Phenole aromatische (isozyklische oder heterozyklische) Verbindungen mit mindestens einer Hydroxygruppe in Betracht kommen. Als Beispiele für die in der Komponente (A2) enthaltenen Amine, Aminonitrobenzole und Phenole können insbesondere genannt werden:
Ethanolamin; Propylamin; 3-Amino-1-propanol; Butylamin; 4-Amino-1 butanol; 1,4-Diamino-benzol (p-Phenylendiamin); 1,4-Diamino-2-methyl-benzol (p-Toluylendiamin); 1,4-Diamino-2,6-dimethyl-benzol; 1,4-Diamino-3,5-diethyl-benzol; 1,4-Diamino-2,5-dimethyl-benzol; 1,4-Diamino-2,3-dimethyl-benzol; 2-Chlor-1,4-diaminobenzol; 2,5-Diaminobiphenyl; 1,4-Diamino-2-hydroxymethyl-benzol; 1,4-Diamino-2-(2-hydroxyethoxy)-benzol; 2-(2-(Acetylamino)ethoxy)-1,4-diamino-benzol; N-Methyl-anilin; 4-Phenylamino-anilin; 4-Dimethylamino-anilin; 4-Diethylamino-anilin; 4-Dipropylamino-anilin; 4-[Ethyl(2-hydroxyethyl)-amino]-anilin; 4-[Di(2'-hydroxy-ethyl)amino]-anilin; 4-[Di(2-hydroxyethyl)-amino]-2-methyl-anilin; 4-[(2'-Methoxyethyl)amino]-anilin; 4-[(3'-Hydroxypropyl)-amino]-anilin; 4-[(2,3-Dihydroxypropyl)amino]-anilin; 1,4-Diamino-2-(2'-hydroxyethyl)-benzol; 1,4-Diamino-2-(1'-hydroxyethyl)-benzol; 1,4-Diamino-2-(1'-methylethyl)-benzol; 1,4-Diamino-2-(methoxy-methyl)-benzol; 1,4-Diamino-2-(aminomethyl)-benzol; 1,3-Bis[(4'-amino-phenyl)(2'-hydroxyethyl)amino]-2-propanol; 1,4-Bis[(4-Aminophenyl)amino]-butan; 1,8-Bis(2',5'-diaminophenoxy)-3,6-dioxaoctan; Phenol; Hydrochinon; 2-Methyl-phenol; 3-Methyl-phenol; 4-Methyl-phenol; 5-Methyl-2-(1'-methylethyl)-phenol; 4-Amino-phenol; 4-Amino-3-methyl-phenol; 4-Amino-3-(hydroxymethyl)-phenol; 4-Amino-3-fluor-phenol; 4-Methylamino-phenol; 4-Amino-2-(aminomethyl)-phenol; 4-Amino-2-(hydroxymethyl)-phenol; 4-Amino-2-fluor-phenol; 4-Amino-2-[(2'hydroxyethyl)-amino]methyl-phenol; 4-Amino-2-methyl-phenol; 4-Amino-2-(methoxymethyl)-phenol; 4-Amino-2-(2'-hydroxyethyl)-phenol; 5-Amino-salicylsäure; 2,5-Diaminopyridin; 2,4,5,6-Tetraamino-pyrimidin; 2,5,6-Triamino-4-(1H)-pyrimidon; 4,5-Diamino-1-((4'-methylbenzyl)-1H-pyrazol; 4,5 Diamino-1H-pyrazol; 4,5-Diamino-1-(4'-methoxybenzyl)-1H-pyrazol; 4,5- Diamino-1-(3'-methoxybenzyl)-1H-pyrazol; 4,5-Diamino-1-(4'-chiorbenzy))-1H-pyrazol; 4,5-Diamino-1-((4'-methylphenyl)-1H-pyrazol; 4,5-Diamino-1-(4'-methoxyphenyl)-1H-pyrazol; 4,5- Diamino-1-(3'-methoxyphenyl)-1H-pyrazol; 4,5-Diamino-1-(4'-chlorphenyl)-1H-pyrazol; 4,5-Diamino-1-(2'-hydroxyethyl)-1H-pyrazol; 4,5-Diamino-1-methyl-1H-pyrazol; 4,5-Diamino-1-ethyl-1H-pyrazol; 4-Amino-1-((4'-methoxyphenyl)-methyl)-5-(methylamino)-1H-pyrazol; 4-Amino-5-((2'-hydroxyethyl)amino)-1-(phenylmethyl)-1H-pyrazol; 4,5-Diamino-1-methyl-3-phenyl-1H-pyrazol; 4,5-Diamino-1-(2'-hydroxyethyl)-3-phenyl-1H-pyrazol; 4,5-Diamino-1,3-dimethyl-1H-pyrazol; 4,5-Diamino-3-methyl-1-phenyl-1H-Pyrazol; 4,5-Diamino-1-(1-isopropyl)-1H-pyrazol; 2-Amino-phenol; 2-Amino-6-methyl-phenol; 2-Amino-5-methyl-phenol; 1,4-Diamino-2-(thiophen-2-yl)benzol; 1,4-Diamino-2-(thiophen-3-yl)-benzol; 1,4-Diamino-2-(pyridin-3-yl)benzol; 1,2,4-Trihydrodroxy-benzol; N-(3-Dimethylamino-phenyl)-hamstoff; 2,6-Diamino-pyridin; 2-Amino-4-[(2'-hydroxyethyl)-amino]-anisol; 2,4-Diamino-1-fluor-5-methyl-benzol; 2,4-Diamino-1-methoxy-5-methyl-benzol; 2,4-Diamino-1-ethoxy-5-methylbenzol; 2,4-Diamino-1-(2'-hydroxyethoxy)-5-methyl-benzol; 2,4-Di[(2'-hydroxyethyl)-amino]-1,5-dimethoxybenzol; 2,3-Diamino-6-methoxypyridin; 3-Amino-6-methoxy-2-(methylamino)-pyridin; 2,6-Diamino-3,5-dimethoxy-pyridin; 3,5-Diamino-2,6-dimethoxy-pyridin; 1,3-Diaminobenzol (m-Phenylen-diamin); 2,4-Diamino-1-(2'-hydroxyethoxy)-benzol; 2,4-Diamino-1,5-di(2'-hydroxyethoxy)-benzol; 1,3-Diamino-4-(2,3-dihydroxypropoxy)-benzol; 1,3-Diamino-4-(3-hydroxypropoxy)-benzol; 1,3-Diamino-4-(2-methoxyethoxy)-benzol; 1-(2'-Aminoethoxy)-2,4-diamino-benzol; 2-Amino-1-(2'-hydroxyethoxy)-4-methylamino-benzol; 2,4-Diaminophenoxyessigsäure; 3-[Di(2'-hydroxyethyl)-amino]-anilin; 4-Amino-2-di[(2'hydroxy-ethyl)amino]-1-ethoxy-benzol; 5-Methyl-2-(1-methylethyl)-phenol; 3-[(2'-Hydroxyethyl)amino]-anilin; 3-[(2'-Aminoethyl)amino]-anilin; 1,3-Di(2',4'-diaminophenoxy)-propan; Di(2',4'-diaminophenoxy)-methan; 1,3-Diamino-2,4-dimethoxy-benzol; 2,6-Bis(2'-hydroxyethyl)amino-toluol; 4-Hydroxyindol; 3-Dimethylaminophenol; 3-Diethylamino-phenol; 5-Amino-2-methyl-phenol; 5-Amino-4-fluor-2-methyl-phenol; 5-Amino-4-methoxy-2-methyl-phenol; 5-Amino-4-ethoxy-2-methyl-phenol; 3-Amino-2,4-dichlorphenol; 5-Amino-2,4-dichlorphenol; 3-Amino-2-methyl-phenol; 3-Amino-2-chlor-6-methyl-phenol; 3-Amino-phenol; N-[(3'-Hydroxyphenyl)-amino]acetamid; 5-[(2-Hydroxyethyl)aminol-2-methyl-phenol; 5-[(2-Hydroxy-ethyl)aminol-4-methoxy-2-methyl-phenol; 3-[(2'-Hydroxyethyl)amino]-phenol; 3-[(2'-Methoxyethyl)-amino]-phenol; 5-Amino-2-ethyl-phenol; 5-Amino-2-methoxy-phenol; 2-(4'-Amino-2'-hydroxyphenoxy)-ethanol; 5-[(3'-Hydroxypropyl)amino]-2-methyl-phenol; 3-[(2',3'-Dihydroxypropyl)-amino]-2-methyl-phenol; 3-[(2'-Hydroxyethyl)-amino]-2-methyl-phenol; 2-Amino-3-hydroxy-pyridin; 2,6-Dihydroxy-3,4-dimethylpyridin; 5-Amino-4-chlor-2-methyl-phenol; 1-Naphthol; 1,5-Dihydroxy-naphthalin; 1,7-Dihydroxy-naphthalin; 2,3-Dihydroxynaphthalin; 2,7-Dihydroxy-naphthalin; 2-Methyl-1-naphtholacetat; 1,3-Dihydroxy-benzol (Resorcin); 1-Chlor-2,4-dihydroxy-benzol; 2-Chlor-1,3-dihydroxy-benzol; 1,2-Dichlor-3,5-dihydroxy-4-methyl-benzol; 1,5-Dichlor-2,4-dihydroxy-benzol; 1,3-Dihydroxy-2-methyl-benzol; 3,4-Methylendioxyphenol; 3,4-Methylendioxy-anilin; 5-[(2'-Hydroxyethyl)-amino]-1,3-benzodioxol; 6-Brom-1-hydroxy-3,4-methylendioxy-benzol; 3,4-Diaminobenzoesäure; 3,4-Dihydro-6-hydroxy-1,4(2H)-benzoxazin; 6-Amino-3,4-dihydro-1,4(2H)-benzoxazin; 3-Methyl-1-phenyl-5-pyrazolon; 5,6-Dihydroxy-indol; 5,6-Dihydroxy-indolin; 5-Hydroxy-indol; 6-Hydroxyindol; 7-Hydroxy-indol und 2,3-Indolindion; 1,4-Bis[2'-hydroxyethyl)-amino]-2-nitro-benzol; 1-(2'-Hydroxyethyl)amino-2-nitro-4-[di(2'-hydroxyethyl)amino]-benzol; 1-Amino-3-methyl-4-[(2'-hydroxyethyl)-amino]-6-nitrobenzol; 4-[Ethyl-(2'-hydroxyethyl)amino]-1-[(2'-hydroxyethyl)-amino]-2-nitrobenzol-hydrochlorid; 4-[Di(2'-hydroxy-ethyl)amino]-1-[(2'-methoxyethyl)-amino]-2-nitrobenzol; 1-[(2',3'-Dihydroxy-propyl)amino]-4-[methyl-(2'-hydroxyethyl)-amino]-2-nitrobenzol; 1-[(2',3'-Dihydroxypropyl)-amino]-4-[ethyl-(2'hydroxyethyl)amino]-2-nitrobenzol-hydrochlorid; 1-(3'-Hydroxypropyl-amino)-4-[di(2'-hydroxy-ethyl)amino]-2-nitrobenzol; 1-Methylamino-4-[methyl-(2',3'-dihydroxy-propyl)amino]-2-nitrobenzol; 2-((4-Amino-2-nitrophenyl)amino)-5-dimethylamino-benzoesäure; 1-(2'-Aminoethyl-amino)-4-[di(2'-hydroxy-ethyl)amino]-2-nitrobenzol; 1-Amino-4-[(2'-hydroxyethyl)-amino]-2-nitro-benzol; 2-Amino-4,6-dinitrophenol; 1,4-Diamino-2-nitrobenzol; 4-Amino-2-nitro-diphenylamin; 1-Amino-4-[di(2'-hydroxyethyl)-amino]-2-nitrobenzol-hydrochlorid; 1-Amino-5-chlor-4-[(2'-hydroxyethyl)-amino]-2-nitrobenzol; 4-Amino-1-[(2'-hydroxyethyl)amino]-2-nitrobenzol; 4-((2'-Hydroxyethyl)-methylamino)-1-(methylamino)-2-nitrobenzol; 1-Amino-4-((2',3'-dihydroxy-propyl)amino)-5-methyl-2-nitrobenzol; 1-Amino-4-(methylamino)-2-nitro-benzol; 4-Amino-2-nitro-1-((prop-2-en-1-yl)amino)-benzol; 4-Amino-3-nitrophenol; 4-[(2'-Hydroxyethyl)amino]-3-nitrophenol]; 4-[(2-Nitrophenyl)amino]phenol; 1-[(2'-aminoethyl)-amino]-4-(2'-hydroxyethoxy)-2-nitrobenzol; 4-(2',3'-Dihydroxypropoxy)-1-[(2'-hydroxy-ethyl)amino]-2-nitrobenzol; 1-Amino-5-chlor-4-[(2',3'-dihydroxypropyl)-amino]-2-nitrobenzol; 5-Chlor-1,4-[di(2',3'dihydroxypropyl)amino] -2-nitrobenzol; 2-[(2'-Hydroxyethyl)-amino]-4,6-dinitro-phenol; 4-Ethylamino-3-nitrobenzoesäure; 2-[(4'-Amino-2'-nitrophenyl)amino]-benzoesäure; 2-Chlor-6-ethylamino-4-nitrophenol; 2-Amino-6-chlor-4-nitrophenol; 4-[(3'-Hydroxypropyl)amino]-3-nitrophenol; 2,5-Diamino-6-nitropyridin; 6-Amino-3-((2'-hydroxyethyl)amino)-2-nitropyridin; 3-Amino-6-((2'-hydroxyethyl)amino)-2-nitropyridin; 3-Amino-6-(ethylamino)-2-nitropyridin; 3-((2'-Hydroxyethyl)amino)-6-(methylamino)-2-nitropyridin; 3-Amino-6-(methylamino)-2-nitropyridin; 6-(Ethylamino)-3-((2'-hydroxyethyl)amino)-2-nitro-pyridin; 1,2,3,4-Tetrahydro-6-nitro-chinoxalin; 7-Amino-3,4-dihydro-6-nitro-2H-1,4-benzoxazin; 1,2-Diamino-4-nitrobenzol; 1-Amino-2-[(2'-hydroxyethyl)amino]-5-nitro-benzol; 1-(2'-Hydroxyethoxy)-2-[(2'-hydroxyethyl)-amino]-5-nitrobenzol; 1-[(2'-Hydroxyethyl)-amino]-2-nitro-benzol; 2-(Di(2'-hydroxyethyl)amino)-5-nitrophenol; 2-[(2'-Hydroxyethyl)-amino]-1-methoxy-5-nitrobenzol; 2-Amino-3-nitrophenol; 1-Amino-2-methyl-6-nitrobenzol; 1-(2'-Hydroxyethoxy)-3-methylamino-4-nitro-benzol; 2,3-(Dihydroxypropoxy)-3-methyl-amino-4-nitrobenzol; 2-[(2'-Hydroxyethyl)-amino]-5-nitrophenol; 3-[(2'-Aminoethyl)-amino]-1-methoxy-4-nitrobenzol-hydrochlorid; 1-[(2'-Ureidoethyl)amino]-4-nitrobenzol; 4-[(2',3'-Dihydroxy-propyl)amino]-3-nitro-1-trifluormethylbenzol; 1-Chlor-2,4-bis[(2'-hydroxy-ethyl)amino]-5-nitrobenzol; 1-Amino-4-((2'-aminoethyl)amino)-5-methyl-2-nitrobenzol; 4-[(2'-Hydroxyethyl)amino]-3-nitro-1-methylbenzol; 1-Chlor-4-[(2'-hydroxyethyl)amino]-3-nitrobenzol; 4-[(2'-Hydroxyethyl)amino]-3-nitro-1-trifluormethylbenzol; 4-[(2'-Hydroxyethyl)amino]-3-nitro-benzonitril; 4-[(2'-Hydroxyethyl)amino]-3-nitrobenzamid; 3-((2'-Hydroxyethyl)amino)-4-methyl-1-nitrobenzol; 4-Chlor-3-((2'-hydroxyethyl)amino)-1-nitrobenzol.

Besonders bevorzugte Amine, Aminonitrobenzole oder Phenole sind Ethanolamin, 1,4-Diaminobenzol; 1,4-Diamino-2-methyl-benzol; 1,4-Diamino-2-(2'-hydroxyethyl)-benzol; 1,4-Diamino-2-(1'-hydroxyethyl)-benzol; 2,4-Diamino-1-(2'-hydroxyethoxy)-benzol; 2,4,5,6-Tetraamino-pyrimidin; Hydrochinon; 5,6-Diamino-2,4-dihydroxy-pyrimidin; 2,7-Diamino-fluoren; 1-Amino-2-naphthol; 2,3-Dihydro-3-methyl-2-benzothiazolon-hydrazon; 2-Aminophenol; 4,5-Diamino-1-(2'-hydroxyethyl)-1H-pyrazol; 4,5-Diamino-1-benzyl-1H-pyrazol; Phenol; 4,5-Diamino-1-((4'-methylbenzyl)-1H-pyrazol; 4,5 Diamino-1H-pyrazol; 4,5-Diamino-1-methyl-1H-pyrazol; 4,5-Diamino-(1-isopropyl)-1H-pyrazol; N-(3-Dimethylamino-phenyl)-harnstoff; 2-Amino-4-[(2'-hydroxyethyl)-amino]anisol; 1,3-Diaminobenzol; 1,3-Di(2,4-diamino-phenoxy)-propan; 4-Methylphenol; 4-Aminophenol; 4-Amino-3-methyl-phenol; 5-Amino-2-methyl-phenol; 3-Aminophenol; 1,3-Dihydroxybenzol; 5-Hydroxyindol; 4-[(2'-hydroxyethyl)-amino]-3-nitro-1-methyl-benzol; 4-[(2'-hydroxyethyl)-amino]-3-nitro-phenol; 2-Amino-6-chlor-4-nitrophenol; 2-Chlor-6-ethylamino-4-nitrophenol; 1-Amino-2-[(2'-hydroxyethyl)amino]-5-nitrobenzol sowie 2,5-Diamino-nitrobenzole, wie zum Beispiel 1-(2'-Hydroxyethyl)-amino-2-nitro-4-[di-(2'-hydroxyethyl)-amino]-benzol, 1-Amino-4-[di(2'-hydroxyethyl)amino]-2-nitrobenzol; 4-[Di(2'-hydroxyethyl)amino]-1-[(2'-methoxy-ethyl)-amino]-2-nitrobenzol, 4-[Ethyl-(2'-hydroxy-ethyl)amino]-1-[(2'-hydroxyethyl)-amino]-2-nitro-benzol, 1-Amino-5-chlor-4-[(2',3'-dihydroxy-propyl)amino]-2-nitrobenzol und 2,5-Diamino-6-nitropyridin.

Die Verbindungen der Formel (I) und die Amine, Aminonitrobenzole oder Phenole werden getrennt voneinander aufbewahrt und erst kurz vor der Anwendung miteinander vermischt. Es ist jedoch auch möglich, sofern die Verbindungen der Formel (I) und die Amine, Aminonitrobenzole und/oder Phenole in fester Form vorliegen, diese gemeinsam abzupacken und das gebrauchsfertige Färbemittel (A) kurz vor der Anwendung durch Vermischen der Verbindungen der Formel (I) und der Amine, Aminonitrobenzole und/oder Phenole mit Wasser oder einer die übrigen Bestandteile des Mittels enthaltenden flüssigen Zubereitung herzustellen.

Weiterhin kann das erfindungsgemäße Färbemittel zusätzlich zu den Verbindungen der Formel (I) sowie den Aminen, Aminonitrobenzolen und Phenolen in der Komponente (A2) sowie der gebrauchsfertigen Zubereitung (A) gegebenenfalls zusätzlich weitere übliche, physiologisch unbedenkliche, direktziehende Farbstoffe aus der Gruppe der kationischen und anionischen Farbstoffe, der Dispersionsfarbstoffe, der Azofarbstoffe, der Chinonfarbstoffe und der Triphenylmethanfarbstoffe enthalten.

Diese direktziehenden Farbstoffe können in der Komponente (A2) in einer Gesamtmenge von etwa 0,02 bis 20 Gewichtsprozent, vorzugsweise 0,2 bis 10 Gewichtsprozent, eingesetzt werden, wobei die Gesamtmenge an direktziehenden Farbstoffen in dem durch Vermischen der Komponenten (A1) und (A2) erhaltenen gebrauchsfertigen Färbemittel (A) etwa 0,01 bis 10 Gewichtsprozent, vorzugsweise 0,1 bis 5 Gewichtsprozent, beträgt.

Das erfindungsgemäße Färbemittel besteht in der Regel aus einer Mischung der Komponenten (A1) und (A2), nämlich einer Farbträgermasse (A1), welche die Verbindung der Formel (I) enthält, und einer weiteren Farbträgermasse (A2), welche die Amine, Aminonitrobenzole und/oder Phenole enthält.

Die Verbindungen der Formel (I) sowie die Amine, Aminonitrobenzole und/oder Phenole sind in der jeweiligen Farbträgermasse (Komponente (A1) bzw. Komponente (A2)) jeweils in einer Gesamtmenge von etwa 0,02 bis 20 Gewichtsprozent, vorzugsweise etwa 0,2 bis 10 Gewichtsprozent, enthalten, wobei in dem gebrauchsfertigen Färbemittel (A) die Verbindungen der Formel (I) sowie die Amine, Aminonitrobenzole und/oder Phenole jeweils in einer Gesamtmenge von etwa 0,01 bis 10 Gewichtsprozent, vorzugsweise etwa 0,1 bis 5 Gewichtsprozent, enthalten sind.

Die Zubereitungsform für die Komponenten (A1) und (A2) sowie des gebrauchsfertigen Färbemittels (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Verbindung der Formel (I) beziehungsweise der Amine, Aminonitrobenzole und/oder Phenole mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylmmoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (jeweils bezogen auf die Komponente (A1) bzw. (A2)), die Verdicker in einer Menge von etwa 0,1 bis 25 Gewichtsprozent (jeweils bezogen auf die Komponente (A1) bzw. (A2)) und die Pflegestoffe in einer Konzentration von etwa 0,1 bis 5,0 Gewichtsprozent (jeweils bezogen auf die Komponente (A1) bzw. (A2)).

Der pH-Wert des gebrauchsfertigen Färbemittels (A) beträgt etwa 3 bis 12, vorzugsweise etwa 4 bis 10, und stellt sich in der Regel bei der Mischung der Komponente (A1) mit der Komponente (A2) ein.
Zur Einstellung des für die Färbung gewünschten pH-Wertes der Komponenten (A1) und (A2) sowie des gebrauchsfertigen Färbemittels (A) können -falls erforderlich- alkalisierende Mittel, wie zum Beispiel Alkalihydroxide, Erdalkalihydroxide, Alkaliacetate, Erdalkaliacetate, Alkalicarbonate oder Erdalkalicarbonate, oder Säuren, wie zum Beispiel Milchsäure, Essigsäure, Weinsäure, Phosphorsäure, Salzsäure, Zitronensäure, Ascorbinsäure oder Borsäure, verwendet werden.

Das gebrauchsfertige Färbemittel wird unmittelbar vor der Anwendung durch Vermischen der Komponenten (A1) und (A2) -gegebenenfalls unter Zusatz von einer Base, wie zum Beispiel Natriumacetat, Natriumbicarbonat oder Natriumcarbonat - hergestellt und sodann auf die Faser, insbesondere menschliche Haare, aufgetragen. Je nach gewünschter Farbtiefe läßt man diese Mischung etwa 5 bis 60 Minuten, vorzugsweise etwa 15 bis 30 Minuten, bei einer Temperatur von etwa 20 bis 50 °C, insbesondere bei etwa 30 bis 40 °C einwirken. Anschließend wird die Faser mit Wasser gespült, gegebenenfalls mit einem Shampoo gewaschen und sodann getrocknet.

Ein weiterer Gegenstand der vorliegenden Erfindung ist ein Mehrkomponenten-Kit, bestehend aus einem Mittel der Komponente (A1), einem Mittel der Komponente (A2), sowie gegebenenfalls einem Mittel zur Einstellung des pH-Wertes. Selbstverständlich können auch die Mittel der Komponenten (A1) und (A2) aus mehreren Einzelkomponenten bestehen, welche erst unmittelbar vor der Anwendung miteinander vermischt werden. Ebenfalls ist ein 2-Komponenten-Kit möglich, dessen erste Komponente aus einem die Verbindungen der Formel (I) sowie die Amine, Aminonitrobenzole und/oder Phenole und gegebenenfalls weitere übliche pulverförmige kosmetische Zusatzstoffe enthaltenden Pulver besteht, und dessen zweite Komponente Wasser oder eine flüssige kosmetische Zubereitung ist. Besonders bevorzugt ist jedoch ein 2-Komponenten-Kit, bestehend aus einem Mittel der Komponente (A1) und einem Mittel der Komponente (A2).

Das erfindungsgemässe Färbemittel ermöglicht eine schonende, gleichmässige und dauerhafte Färbung der Fasern, insbesondere von Keratinfasern, wie zum Beispiel menschlichen Haaren, wobei eine breite Farbpalette von gelben bis dunkelvioletten beziehungsweise braunschwarzen Farbtönen möglich ist.

Aufgrund ihrer guten Stabilität gegenüber Oxidationsmitteln können die Verbindungen der allgemeinen Formel (I) auch in Oxidationsfärbemitteln auf der Basis von Oxidationsfarbstoffvortsufen verwendet werden. Ebenfalls ist es möglich, die Verbindungen der allgemeinen Formel (I) in sogenannten aufhellenden Tönungen zu verwenden, bei denen -zwecks Aufhellung der zu färbenden Haare oder zur Glanzverbesserung- die direktziehenden Farbstoffe in Kombination mit einem Oxidationsmittel eingesetzt werden.

Die nachfolgenden Beispiele sollen den Gegenstand näher erläutern, ohne ihn auf diese Beispiele zu beschränken.

### Beispiele

### Beispiele 1 bis 21: Haarfärbemittel

### Komponente (A1)

| | |
|---|---|
| 0,5 g | 7-Chloro-4-nitro-2,1,3-benzoxadiazol |
| 5,0 g | Ethanol |
| 4,0 g | Decylpolyglucosid (wässrige Lösung ; Plantaren® 2000 der Firma Cognis, Deutschland), |
| 0,2 g | Ethylendiaminotetraessigsäure-Dinatriumsalz-Hydrat |
| ad 100,0 g | Wasser, vollentsalzt |

### Komponente (A2)

X g Amin und/oder Phenol in Pulverform gemäß Tabelle 1.

Bei Raumtemperatur (20-25 °C) oder unter leichtem Erwärmen (35-40 °C) wird die vorstehend genannte Mischung (Komponente (A1)) mit dem das Amin oder Phenol enthaltenden Pulver (Komponente (A2)) - gegebenenfalls unter Zusatz einer Spatelspitze Natriumacetat- homogen vermischt. Die pH-Werte werden -falls erforderlich- mit Natronlauge oder Zitronensäure auf den gewünschten Wert eingestellt.

Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit lauwarmem Wasser gespült und sodann getrocknet.

Die Einsatzmenge des Amins oder Phenols sowie die erhaltenen Färbungen sind in der nachfolgenden Tabelle 1 zusammengefasst.

**Tabelle 1:**

| **Beispiel Nr.** | **verwendetes Amin bzw. Phenol (Menge in g)** | **pH-Wert** | **Farbton nach dem Färben** | **Farbmeßwerte** | | |
|---|---|---|---|---|---|---|
| | | | | **L** | **a** | **b** |
| **1** | Ethanolamin (0,153g) | 9,3 | gelb | +41,16 | +11,42 | +43,41 |
| **2** | Phenol (0,235 g) | 3,5 | gelb | +57,82 | + 8,43 | +62,90 |
| **3** | Hydrochinon (0,275 g) | 7,0 | orange | +69,52 | + 2,72 | +46,43 |
| **4** | 4-Dimethylaminoanilin (0,340 g) | 4,6 | violett | +31,83 | +13,15 - | 2,80 |
| **5** | 1,4-Diaminobenzol (0,270 g) | 4,7 | violett | +26,36 | +23,37 - | 0,48 |
| **6** | 1,4-Diamino-2-methyl-benzol•HCl (0,055 g) | 4,5 | violett | +30,81 | +21,60 | + 0,28 |
| **7** | 1,4-Diamino-2-(2-hydroxyethyl)-benzol sulfat (0,630 g) | 3,9 | violett | +21,33 | +17,74 | + 2,16 |
| **8** | 4-Aminophenol (0,273 g) | 3,4 | rubinrot | +26,13 | +39,62 | +15,48 |
| **9** | 2,4,5,6-Tetraamino-pyrimidin-sulfat (0,640 g) | 9,8 | grünbraun | +34,85 | +4,30 | +18,31 |
| **10** | 5,6-Diamino-2,4-dihydroxy-pyrimidin-sulfat (0,455 g) | 7,3 | braunorange | +32,73 | +15,18 | +28,79 |
| **11** | 4,5-Diamino-1-(2'-hydroxyethyl)-1H-pyrazol-sulfat (0,600 g) | 9,3 | rot | +29,07 | +38,14 | +22,13 |
| **12** | 2,7-Diamino-fluoren (0,490 g) | 7,4 | violett | +26,95 | +18,76 | +5,13 |
| **13** | 1-Amino-2-naphthol•HCl (0,489 g) | 6,3 | braun | +41,520 | + 7,03 | +20,43 |
| **14** | 2,3-Dihydro-3-methyl-2-benzothiazolon-hydrazon•HCl (0,584 g) | 6,4 | orange | +44,00 | +40,62 | +46,52 |
| **15** | N-(3-Dimethyl-amino-phenyl)-harnstoff (0,448 g) | 6,5 | gelbbraun | +35,42 | + 1,75 | +14,81 |
| **16** | 2-Amino-4-[(2'-hydroxyethyl)amino]-anisol-sulfat (0,746 g) | 6,7 | grau | +47,79 | -2,78 | +1,60 |
| **17** | 1,3-Diaminobenzol (0,270 g) | 4,3 | rotviolett | +32,31 | +18,33 | +11,25 |
| **18** | 2,4-Diamino-1-(2'-hydroxyethoxy)-benzol-sulfat (0,666 g) | 4,1 | blauviolett | +26,41 | +4,77 | -2,69 |
| **19** | 5-Amino-2-methyl-phenol (0,308 g) | 4,5 | rubinrot | +32,96 | +39,20 | +24,27 |
| **20** | 3-Amino-phenol (0,273 g) | 4,4 | rubinrot | +38,98 | +40,37 | +34,32 |
| **21** | 1,3-Dihydroxy-benzol (0,275 g) | 4,0 | gelb | +70,64 | +8,37 | +47,07 |

### Beispiele 22 bis 28 : Haarfärbemittel

### Komponente (A1):

| | |
|---|---|
| 1,0 g | 7-Chloro-4-nitro-2,1,3-benzoxadiazol |
| 5,0 g | Ethanol |
| 4,0 g | Decylpolyglucose (Plantaren® 2000), wässrige Lösung |
| 0,2 g | Ethylendiaminotetraessigsäure-Dinatriumsalz-Hydrat |
| ad 100,0 g | Wasser, vollentsalzt |

### Komponente (A2):

| | |
|---|---|
| X g | Amin oder Phenol gemäß Tabelle 2 |
| 5,0 g | Ethanol |
| 4,0 g | Decylpolyglucose (Plantaren® 2000), wässrige Lösung |
| 0,2 g | Ethylendiaminotetraessigsäure-Dinatriumsalz-Hydrat |
| ad 100,0 g | Wasser, vollentsalzt |

10 g der Komponente (A1) werden mit 10 g der Komponente (A2) bei Raumtemperatur (20-25 °C) oder unter leichtem Erwärmen (35-40 °C) -gegebenenfalls unter Zusatz einer Spatelspitze Natriumacetat- homogen miteinander vermischt. Die pH-Werte werden -falls erforderlich- mit Natronlauge oder Zitronensäure auf den gewünschten Wert eingestellt.

Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit lauwarmem Wasser gespült und sodann getrocknet.

Die Einsatzmenge des Amins und/oder Phenols sowie die erhaltenen Färbungen sind in der nachfolgenden Tabelle 2 zusammengefasst.

**Tabelle 2:**

| **Beispiel Nr.** | **verwendetes Amin bzw. Phenol (Menge in g)** | **pH-Wert** | **Farbton nach dem Färben** | **Farbmeßwerte** | | |
|---|---|---|---|---|---|---|
| | | | | **L** | **a** | **b** |
| **22** | 1,4-Diaminobenzol (0,540 g) | 4,8 | | +21,77 | +20,65 | + 0,80 |
| | | 6,8 | violett | +26,66 | +25,47 - | 1,39 |
| | | 9,8 | | +30,48 | +24,70 - | 0,35 |
| **23** | 1,4-Diamino-2-methyl-benzol-sulfat (1,100 g) | 4,7 | | +21,69 | +19,99 | + 3,57 |
| | | 6,8 | violett | +29,24 | +22,23 | + 5,84 |
| | | 10,0 | | +30,14 | +19,04 | + 4,62 |
| **24** | 4-Aminophenol (0,546 g) | 4,6 | | +23,87 | +35,97 | +13,01 |
| | | 6,9 | rubinrot | +25,30 | +39,15 | +14,64 |
| | | 9,5 | | +23,31 | +35,70 | +12,80 |
| **25** | 2-Aminophenol (0,546 g) | 4,8 | | +34,39 | +40,22 | +29,97 |
| | | 6,6 | rotorange | +34,59 | +39,94 | +30,89 |
| | | 9,2 | | +32,15 | +34,18 | 38,30 |
| **26** | Phenol (0,470 g) | 4,7 | | +67,34 | + 6,41 | +76,05 |
| | | 6,8 | gelb | +62,29 | + 9,85 | +73,75 |
| | | 9,8 | | +68,61 | + 4,33 | +70,47 |
| **27** | Phenol (0,234 g) und 1,4-Diamino-2-methyl-benzol-sulfat (0,550 g) | 4,3 | | +43,82 | +22,25 | +12,60 |
| | | 7,7 | rosa-violett | +47,73 | +20,71 | + 8,74 |
| | | 10,0 | | 37,17 | +20,80 | + 2,67 |
| **28** | 2-Aminophenol (0,272 g) und 4-Aminophenol (0,272 g) | 4,4 | | +23,96 | +35,28 | +13,08 |
| | | 7,7 | rubinrot | +21,82 | +30,59 | +10,46 |
| | | 9,5 | | +21,95 | +27,56 | +10,03 |

### Beispiele 29 und 30 : Haarfärbemittel in Cremeform

### Komponente (A1):

| | |
|---|---|
| 1 g | 7-Chloro-4-nitro-2,1,3-benzoxadiazol |
| 12 g | Cetearylalkohol |
| 10 g | Laurylethersulfat, 28%ige wässrige Lösung |
| 20 g | Ethanol |
| ad 100 g | Wasser, vollentsalzt |

Der Cetearylalkohol wird bei 80 °C geschmolzen. Das Laurylethersulfat wird mit 95% des Wasser auf 80 °C erhitzt und zum geschmolzenen Cetearylalkohol gegeben und gerührt bis sich eine Creme bildet. Bei Raumtemperatur wird das 7-Chloro-4-nitro-2,1,3-benzoxadiazol mit dem Ethanol und dem restlichen Wasser vermischt und der Creme zugegeben.

Der pH Wert der so erhaltenen Creme beträgt 4,6.

### Komponente (A2)

| | |
|---|---|
| X g | Amin oder Phenol gemäß Tabelle 3 |
| 12 g | Cetearylalkohol |
| 10 g | Laurylethersulfat, 28%ige wässrige Lösung |
| 20 g | Ethanol |
| ad 100 g | Wasser, vollentsalzt |

Der Cetearylalkohol wird bei 80 °C geschmolzen. Das Laurylethersulfat wird mit 95% des Wasser auf 80 °C erhitzt und zum geschmolzenen Cetearylalkohol gegeben und gerührt bis sich eine Creme ergibt. Bei Raumtemperatur wird das Amin oder Phenol mit dem Ethanol und dem restlichen Wasser vermischt und zur Creme gegeben. Der pH Wert der so erhaltenen Creme wird (gegebenfalls unter Zusatz von Natriumacetat) auf 7 eingestellt.

Die Komponenten (A1) und (A2) werden im Verhältnis 1:1 miteinander vermischt. Gegebenfalls wird eine Spatelspitze Natriumacetat dazugegeben. Der pH-Wert des gebrauchsfertigen Färbemittels wird -falls erforderlich- mit Natronlauge oder Zitronensäure auf den gewünschten Wert eingestellt. Das so erhaltene gebrauchsfertige Haarfärbemittel wird auf die Haare aufgetragen und mit einem Pinsel gleichmäßig verteilt. Nach einer Einwirkungszeit von 30 Minuten bei 40 °C wird das Haar mit einem Shampoo gewaschen, anschließend mit lauwarmem Wasser gespült und sodann getrocknet.

Die Einsatzmenge des Amins oder Phenols sowie die erhaltenen Färbungen sind in der nachfolgenden Tabelle 3 zusammengefasst.

**Tabelle 3:**

| **Beispiel Nr.** | **verwendetes Amin bzw. Phenol (Menge in g)** | **pH-Wert** | **Farbton nach dem Färben** | **Farbmeßwerte** | | |
|---|---|---|---|---|---|---|
| | | | | **L** | **a** | **b** |
| **29** | 1,4-Diaminobenzol (1,350 g) | 7,2 | violett | +20,8 | +20,3 | + 1,4 |
| **30** | 4-Aminophenol (1,370 g) | 7,3 | rubinrot | +22,4 | +35,7 | +11,4 |

### Beispiele 31 bis 42 : Haarfärbemittel

### Komponente (A1):

| | |
|---|---|
| 1,0 g | 7-Chloro-4-nitro-2,1,3-benzoxadiazol |
| 5,0 g | Ethanol |
| 4,0 g | Decylpolyglucosid (wässrige Lösung; Plantaren® 2000 der Firma Cognis, Deutschland), |
| 0,2 g | Ethylendiaminotetraessigsäure-Dinatriumsalz-Hydrat |
| ad 100,0 g | Wasser, vollentsalzt |

### Komponente (A2)

| | |
|---|---|
| X g | Aminonitrobenzol gemäß Tabelle 4 |
| 5,0 g | Ethanol |
| 4,0 g | Decylpolyglucose (Plantaren® 2000), wässrige Lösung |
| 0,2 g | Ethylendiaminotetraessigsäure-Dinatriumsalz-Hydrat |
| ad 100,0 g | Wasser, vollentsalzt |

10 g der Komponente (A1) werden mit 10 g der Komponente (A2) bei Raumtemperatur (20-25 °C) oder unter leichtem Erwärmen (35-40 °C) -gegebenenfalls unter Zusatz einer Spatelspitze Natriumacetat- homogen miteinander vermischt, wobei die Mischung erforderlichernfalls für 15 Minuten auf 40 bis 60 °C erwärmt werden kann. Die pH-Werte werden -falls erforderlich- mit Natronlauge oder Zitronensäure auf den gewünschten Wert eingestellt.

Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit lauwarmem Wasser gespült und sodann getrocknet.

Die Einsatzmenge des Aminonitrobenzols sowie die erhaltenen Färbungen sind in der nachfolgenden Tabelle 4 zusammengefasst.

**Tabelle 4:**

| **Beispiel Nr.** | **verwendetes Aminonitrobenzol (Menge in g)** | **pH-Wert** | **Farbton nach dem Färben** | **Farbmeßwerte** | | |
|---|---|---|---|---|---|---|
| | | | | **L** | **a** | **b** |
| **31** | 1-(2'-Hydroxyethyl)-amino-2-nitro-4[di-(2'-hydroxyethyl)-amino]-benzol (1,420 g) | 6,3 | raunschwarz mit violetten Reflexen | +17,19 | +3,94 | + 2,43 |
| **32** | 4-[Di(2'-hydroxy-ethyl)amino]-1-[(2-methoxyethyl)-amino]-2-nitro-benzol (1,490 g) | 4,9 | raunschwarz mit violetten Reflexen | +18,47 | + 5,56 | + 3,59 |
| **33** | 4-[Ethyl-(2'-hydroxy-ethyl)amino]-1-[(2'-hydroxyethyl)-amino]-2-nitro-benzol•HCl (1,520 g) | 5,4 | raunschwarz mit violetten Reflexen | +18,27 | + 3,45 | + 2,71 |
| **34** | 1-Amino-4-[di(2'-hydroxyethyl)amino] -2- nitro-benzol•HCl (1,200 g) | 5,2 | braun mit roten Reflexen | +18,10 | +10,07 | +3,93 |
| **35** | 1-Amino-5-chlor-4-[(2',3'-dihydroxy-propyl)amino]-2-nitrobenzol (1,300 g) | 7,0 | kirschrot | +20,35 | +24,84 | +7,43 |
| **36** | 1-Amino-2-[(2'-hydroxyethyl)amino] -5-nitrobenzol (0,980 g) | 10,0 | rotorange | +29,98 | +34,15 | +23,20 |
| **37** | 4-[(2'-hydroxyethyl)-amino]-3-nitro-1-methyl-benzol (0,980 g) | 9,4 | orange | +51,18 | +30,12 | +59,68 |
| **38** | 4-[(2'-hydroxyethyl)-amino]-3-nitro-phenol (0,990 g) | 6,2 | rubinrot | +30,91 | +48,81 | +25,69 |
| **39** | 2-Amino-6-chlor-4-nitrophenol•HCl (1,120 g) | 4,7 | braunviolett | +17,74 | +15,06 | + 4,19 |
| **40** | 2-Chlor-6-ethyl-amino-4-nitrophenol (1,080 g) | 7,5 | rot | +29,73 | +38,89 | +23,40 |
| **41** | 2,5-Diamino-6-nitropyridin (0,770 g) | 6,5 | orange | +39,10 | 37,92 | +40,11 |
| **42** | 1-Amino-4-[di(2'-hydroxyethyl)amino] -2-nitrobenzol•HCl (0,60 g) und 4-[Di-(2'-hydroxy-ethyl)-amino]-1-[(2'-methoxyethyl)-amino]-2-nitrobenzol (0,750 g) | 4,8 | braun mit roten Reflexen | +18,76 | + 9,46 | + 3,08 |

### Beispiele 43 bis 51: Haarfärbemittel

### Komponente (A1)

| | |
|---|---|
| 0,488 g | 7-Methoxy-4-nitro-2,1,3-benzoxadiazol |
| 5,0 g | Ethanol |
| 4,0 g | Decylpolyglucosid (wässrige Lösung; Plantaren® 2000 der Firma Cognis, Deutschland), |
| 0,2 g | Ethylendiaminotetraessigsäure-Dinatriumsalz-Hydrat |
| ad 100,0 g | Wasser, vollentsalzt |

### Komponente (A2)

X g Amin und/oder Phenol in Pulverform gemäß Tabelle 5.

Bei Raumtemperatur (20-25 °C) oder unter leichtem Erwärmen (35-40 °C) wird die vorstehend genannte Mischung (Komponente (A1)) mit dem das Amin oder Phenol enthaltenden Pulver (Komponente (A2)) - gegebenenfalls unter Zusatz einer Spatelspitze Natriumacetat- homogen vermischt. Die pH-Werte werden -falls erforderlich- mit Natronlauge oder Zitronensäure auf den gewünschten Wert eingestellt.

Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit lauwarmem Wasser gespült und sodann getrocknet.

Die Einsatzmenge des Amins oder Phenols sowie die erhaltenen Färbungen sind in der nachfolgenden Tabelle 5 zusammengefasst.

**Tabelle 5:**

| **Beispiel Nr.** | **verwendetes Amin bzw. Phenol (Menge in g)** | **pH-Wert** | **Farbton nach dem Färben** | **Farbmeßwerte** | | |
|---|---|---|---|---|---|---|
| | | | | **L** | **a** | **b** |
| **43** | 1,4-Diamino-2-methyl-benzol-sulfat (0.55g) | 4,7 | violett | +21,82 | +19,70 | + 2,28 |
| **44** | 1,4-Diamino-2-(2-hydroxyethyl)-benzol-sulfat (0, 63 g) | 4,6 | violett | +17,75 | +11,85 | +1,25 |
| **45** | 4-Aminophenol (0,273 g) | 7,6 | rubinrot | +31,05 | +46,43 | +20,06 |
| **46** | 4-Amino-3-methyl-phenol | 9,3 | orange | +44,84 | +32,42 | +43,04 |
| **47** | 4,5-Diamino-1-(2'-hydroxyethyl)-1H-pyrazol-sulfat (0,600 g) | 9,9 | rot | +28,26 | +34,16 | +17,99 |
| **48** | 2,4-Diamino-1-(2'-hydroxyethoxy)-benzol-sulfat (0,660 g) | 4,4 | rot-violett | +28,41 | + 31,66 | 11,88 |
| **49** | 5-Amino-2-methyl-phenol (0,308 g) | 4,7 | rot | +46,23 | +48,47 | +28,73 |
| **50** | 3-Amino-phenol (0,273 g) | 4,2 | rot-orange | +44,08 | +51,80 | +44,28 |
| **51** | 1,3-Dihydroxy-benzol (0,275 g) | 4,8 | gelb | +78,59 | -0,89 | +62,74 |

Die in den vorliegenden Beispielen angegebenen L*a*b*-Farbmesswerte wurden mit einem Farbmessgerät der Firma Minolta, Typ Chromameter II, ermittelt. Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Alle Prozentangaben in der vorliegenden Anmeldung stellen, sofern nicht anders angegeben, Gewichtsprozente dar.

## Patentansprüche

1. Mittel zur Färbung von Fasern (A), welches durch Vermischen zweier Komponenten (A1) und (A2) hergestellt wird und **dadurch gekennzeichnet ist, dass** die Komponente (A1) mindestens eine Verbindung der Formel (I) enthält, 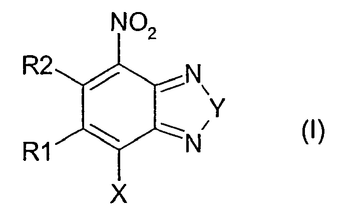 worin X gleich einem Halogenatom, einer Methoxy-Gruppe oder einer Ethoxy-Gruppe ist; Y gleich einem Sauerstoffatom, einem Schwefelatom oder einem Selenatom ist; und R1 und R2 gleich oder verschieden sein können und unabhängig voneinander Wasserstoff, ein Halogenatom, eine (C₁-C₄)-Alkylgruppe, eine mit einem Halogenatom substituierte (C₁-C₄)-Alkylgruppe, eine (C₁-C₄)-Alkoxygruppe, eine Nitrogruppe, eine Acetamidogruppe oder eine NR^{a}R^{b}-Gruppe, wobei die Reste R^{a} und R^{b} gleich oder verschieden sein können und unabhängig voneinander Wasserstoff, eine (C₁-C₄)-Alkylgruppe, einen gegebenfalls substituierten aromatischen Carbozyklus oder eine (C₁-C₄)-Alkancarbonylgruppe darstellen, oder R^{a} und R^{b} gemeinsam mit dem Stickstoffatom einen heterozyklischen (C₃-C₆)- Rest bilden;
und die Komponente (A2) mindestens eine Verbindung aus der Gruppe bestehend aus Aminen, Aminonitrobenzolen und Phenolen enthält.

2. Mittel nach Anspruch 1, **dadurch gekennzeichnet, dass** die Verbindung der Formel (I) ausgewählt ist aus 4-Nitro-2,1,3-benzoxadiazole, 4-Nitro-2,1,3-benzothiadiazole und 4-Nitro-2,1,3-benzoselenadiazole zu nennen: 4-Chloro-7-nitro-2,1,3-benzoxa-diazol; 4-Bromo-7-nitro-2,1,3-benzoxadiazol; 4-Fluoro-7-nitro-2,1,3-benzoxadiazol; 4-Methoxy-7-nitro-2,1,3-benzoxadiazol; 4-Ethoxy-7-nitro-2,1,3-benzoxadiazol; 4-Chloro-5,7-dinitro2,1,3-benzoxadiazol; 5,7-Dichloro-4-nitro-2,1,3-benzoxadiazol; 5,7-Dibromo-4-nitro-2,1,3-benzoxadiazol; 7-Chloro-4-nitro-5-diethylamino-2,1,3-benzoxadiazol; 5-Amino-7-chloro-4-nitro-2,1,3-benzoxadiazol; 7-Chloro-4-nitro-5-(1-piperidinyl)-2,1,3-benzoxadiazol; 4-Chloro-7-nitro- 2,1,3-benzothiadiazol; 4-Bromo-7-nitro-2,1,3-benzothiadiazol; 4-Methoxy-7-nitro-2,1,3-benzothiadiazol; 4-Ethoxy-7-nitro-2,1,3-benzoxathiazol; 4-Chloro-5,7-dinitro-2,1,3-benzothiadiazol; 4-Bromo-5,7-dinitro-2,1,3-benzothiadiazol; 4,5-Dichloro-7-nitro-2,1,3-benzothiadiazol; 5,7-Dichloro-4-nitro-2,1,3-benzothiadiazol; 4,5-Dibromo-7-nitro-2,1,3-benzothiadiazol; 5,7-Dibromo-4-nitro-2,1,3-benzothiadiazol; 4,6-Dichloro-5,7-dinitro-2,1,3-benzothiadiazol; 4,6-Dibromo-5,7-dinitro-2,1,3-benzothiadiazol; 5-Chloro-7-fluoro-4-nitro-2,1,3-benzothiadiazol; 5-Anilino-7-bromo-4-nitro-2,1,3-benzothiadiazol; 5-Amino-7-chloro-4-nitro2,1,3-benzothiadiazol; 7-Bromo-5-(diethylamino)-4-nitro-2,1,3-benzothiadiazol; 4-Chloro-5-methyl-7-nitro-2,1,3-benzothiadiazol; 4-Bromo-5-methyl-4-nitro-2,1,3-benzothiadiazol; 7-Bromo-5-(bromomethyl)-7-nitro-2,1,3-benzothiadiazol; N-(7-Chloro-4-nitro-2,1,3-benzothiadiazol-5-yl)-acetamid; 4-Chloro-7-nitro-2,1,3-benzo-selenadiazol; 4-Bromo-7-nitro-2,1,3-benzoselenadiazol; 4-Methoxy-7-nitro-2,1,3-benzoselenadiazol; 4-Ethoxy-7-nitro-2,1,3-benzoselenadiazol; 5,7-Dibromo-4-nitro-2,1,3-benzoselenadiazol; 5,7-Dichloro-4-nitro-2,1,3-benzoselenadiazol; 7-Bromo-5-methyl-4-nitro-2,1,3-benzoselenadiazol und 7-Bromo-5-(bromomethyl)-4-nitro-2,1,3-benzo-selenadiazol.

3. Mittel nach Anspruch 1, **dadurch gekennzeichnet, dass** das Amin, Aminonitrobenzol und Phenol ausgewählt ist aus Ethanolamin, 1,4-Diaminobenzol; 1,4-Diamino-2-methyl-benzol; 1,4-Diamino-2-(2'-hydroxyethyl)-benzol; 1,4-Diamino-2-(1'-hydroxyethyl)-benzol; 2,4-Diamino-1-(2'-hydroxyethoxy)-benzol; 2,4,5,6-Tetraamino-pyrimidin; Hydrochinon; 5,6-Diamino-2,4-dihydroxy-pyrimidin; 2,7-Diamino-fluoren; 1-Amino-2-naphthol; 2,3-Dihydro-3-methyl-2-benzothiazolon-hydrazon; 2-Aminophenol; 4,5-Diamino-1-(2'-hydroxy-ethyl)-1H-pyrazol; 4,5-Diamino-1-benzyl-1H-pyrazol; Phenol; 4,5-Diamino-1-((4'-methylbenzyl)-1H-pyrazol; 4,5 Diamino-1H-pyrazol; 4,5-Diamino-1-methyl-1H-pyrazol; 4,5-Diamino-(1-isopropyl)-1H-pyrazol; N-(3-Dimethylaminophenyl)-harnstoff; 2-Amino-4-[(2'-hydroxyethyl)-amino]anisol; 1,3-Diaminobenzol; 1,3-Di(2,4-diamino-phenoxy)-propan; 4-Methylphenol; 4-Aminophenol; 4-Amino-3-methyl-phenol; 5-Amino-2-methylphenol; 3-Aminophenol; 1,3-Dihydroxybenzol; 5-Hydroxyindol; 4-[(2'-hydroxyethyl)-amino]-3-nitro-1-methyl-benzol; 4-[(2'-hydroxyethyl)-amino]-3-nitro-phenol; 2-Amino-6-chlor-4-nitrophenol; 2-Chlor-6-ethylamino-4-nitrophenol; 1-Amino-2-[(2'-hydroxyethyl)amino]-5-nitrobenzol sowie 2,5-Diamino-nitrobenzole, wie zum Beispiel 1-(2'-Hydroxyethyl)-amino-2-nitro-4-[di-(2'-hydroxyethyl)-amino]-benzol, 1-Amino-4-[di(2'-hydroxyethyl)amino]-2-nitrobenzol; 4-[Di(2'-hydroxyethyl)amino]-1-[(2'-methoxy-ethyl)-amino]-2-nitrobenzol, 4-[Ethyl-(2'-hydroxy-ethyl)amino]-1-[(2'-hydroxyethyl)-amino]-2-nitro-benzol, 1-Amino-5-chlor-4-[(2',3'-dihydroxy-propyl)amino]-2-nitrobenzol und 2,5-Diamino-6-nitropyridin.

4. Mittel nach einem der Ansprüche 1 bis 3, **dadurch gekennzeichnet, dass** es die Verbindungen der Formel (I) sowie die Amine, Aminonitrobenzole und/oder Phenole in der jeweiligen Farbträgermasse (Komponente (A1) bzw. Komponente (A2)) jeweils in einer Gesamtmenge von 0,02 bis 20 Gewichtsprozent enthält.

5. Mittel nach einem der Ansprüche 1 bis 4, **dadurch gekennzeichnet, dass** es die Verbindungen der Formel (I) sowie die Amine, Aminonitrobenzole und/oder Phenole in dem gebrauchsfertigen Färbemittel (A) jeweils in einer Gesamtmenge von 0,01 bis 10 Gewichtsprozent enthält.

6. Mittel nach einem der Ansprüche 1 bis 5, **dadurch gekennzeichnet, dass** es zusätzlich 0,02 bis 20 Gewichtsprozent eines physiologisch unbedenklichen, direktziehenden Farbstoffs aus der Gruppe der kationischen und anionischen Farbstoffe, der Dispersionsfarbstoffe, der Nitrofarbstoffe, der Azofarbstoffe, der Chinonfarbstoffe und der Triphenylmethanfarbstoffe enthält.

7. Mittel nach einem der Ansprüche 1 bis 6, **dadurch gekennzeichnet, dass** das gebrauchsfertige Färbemittel (A) einen pH-Wert von 3 bis 12 aufweist.

8. Mittel nach einem der Ansprüche 1 bis 7, **dadurch gekennzeichnet, dass** es zusätzlich Oxidationsfarbstoffvorstufen enthält.

9. Mittel nach einem der Ansprüche 1 bis 8, **dadurch gekennzeichnet, dass** es vor der Anwendung mit einem Oxidationsmittel vermischt wird.

10. Mittel nach einem der Ansprüche 1 bis 9, **dadurch gekennzeichnet, dass** es ein Haarfärbemittel ist.

11. Verfahren zum Färben von Haaren bei dem das gebrauchsfertige Färbemittel (A) unmittelbar vor der Anwendung durch Vermischen zweier Komponenten (A1) und (A2) -gegebenenfalls unter Zusatz von Natriumacetat- hergestellt und sodann auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird, **dadurch gekennzeichnet, dass** ein durch Vermischen zweier Komponenten (A1) und (A2) erhältliches Färbemittel (A) gemäß einem der Ansprüche 1 bis 10 verwendet wird.

12. Mehrkomponenten-Kit zum Färben von Haaren, bestehend aus einem Mittel der Komponente (A1) gemäß einem der Ansprüche 1 bis 10 und einem Mittel der Komponente (A2) gemäß einem der Ansprüche 1 bis 10, sowie gegebenenfalls einem Mittel zur Einstellung des pH-Wertes.

13. Verwendung einer Kombination aus mindestens eine Verbindung der Formel (I), 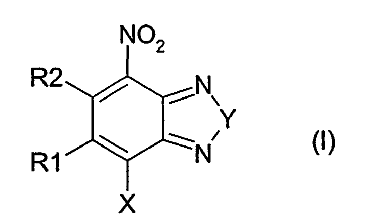 worin X gleich einem Halogenatom, einer Methoxy-Gruppe oder einer Ethoxy-Gruppe ist; Y gleich einem Sauerstoffatom, einem Schwefelatom oder einem Selenatom ist; und R1 und R2 gleich oder verschieden sein können und unabhängig voneinander Wasserstoff, ein Halogenatom, eine (C₁-C₄)-Alkylgruppe, eine mit einem Halogenatom substituierte (C₁-C₄)-Alkylgruppe, eine (C₁-C₄)-Alkoxygruppe, eine Nitrogruppe, eine Acetamidogruppe oder eine NR^{a}R^{b}-Gruppe, wobei die Reste R^{a} und R^{b} gleich oder verschieden sein können und unabhängig voneinander Wasserstoff, eine (C₁-C₄)-Alkylgruppe, einen gegebenfalls substituierten aromatischen Carbozyklus oder eine (C₁-C₄)-Alkancarbonylgruppe darstellen, oder R^{a} und R^{b} gemeinsam mit dem Stickstoffatom einen heterozyklischen (C₃-C₆)-Rest bilden; und mindestens einer Verbindung aus der Gruppe bestehend aus Aminen, Aminonitrobenzolen und Phenolen zur Färbung von Keratinfasern.

## Claims

1. Agent for colouring fibres (A), which is prepared by mixing two components (A1) and (A2) and is **characterized in that** component (A1) comprises at least one compound of the formula (I), 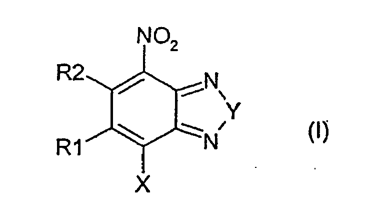 in which X is a halogen atom, a methoxy group or an ethoxy group; Y is an oxygen atom, a sulphur atom or a selenium atom; and R1 and R2 may be identical or different and, independently of one another, are hydrogen, a halogen atom, a (C₁-C₄)-alkyl group, a (C₁-C₄)-alkyl group substituted by a halogen atom, a (C₁-C₄)-alkoxy group, a nitro group, an acetamido group or an NR^{a}R^{b} group, where the radicals R^{a} and R^{b} may be identical or different and, independently of one another, are hydrogen, a (C₁-C₄)-alkyl group, an optionally substituted aromatic carbocycle or a (C₁-C₄)-alkanecarbonyl group, or R^{a} or R^{b}, together with the nitrogen atom, form a heterocyclic (C₃-C₆) radical; and component (A2) comprises at least one compound from the group consisting of amines, aminonitrobenzenes and phenols.

2. Agent according to Claim 1, **characterized in that** the compound of the formula (I) is chosen from 4-nitro-2,1,3-benzoxadiazoles, 4-nitro-2,1,3-benzothiadiazoles and 4-nitro-2,1,3-benzoselenadiazoles: 4-chloro-7-nitro-2,1,3-benzoxadiazole; 4-bromo-7-nitro-2,1,3-benzoxadiazole; 4-fluoro-7-nitro-2,1,3-benzoxadiazole; 4-methoxy-7-nitro-2,1,3-benzoxadiazole; 4-ethoxy-7-nitro-2,1,3-benzoxadiazole, 4-chloro-5,7-dinitro-2,1,3-benzoxadiazole; 5,7-dichloro-4-nitro-2,1,3-benzoxadiazole; 5,7-dibromo-4-nitro-2,1,3-benzoxadiazole; 7-chloro-4-nitro-5-diethylamino-2,1,3-benzoxadiazole; 5-amino-7-chloro-4-nitro-2,1,3-benzoxadiazole; 7-chloro-4-nitro-5-(1-piperidinyl)-2,1,3-benzoxadiazole; 4-chloro-7-nitro-2,1,3-benzothiadiazole; 4-bromo-7-nitro-2,1,3-benzothiadiazole; 4-methoxy-7-nitro-2,1,3-benzothiadiazole; 4-ethoxy-7-nitro-2,1,3-benzoxathiazole; 4-chloro-5,7-dinitro-2,1,3-benzothiadiazole; 4-bromo-5,7-dinitro-2,1,3-benzothiadiazole; 4,5-dichloro-7-nitro-2,1,3-benzothiadiazole; 5,7-dichloro-4-nitro-2,1,3-benzothiadiazole; 4,5-dibromo-7-nitro-2,1,3-benzothiadiazole; 5,7-dibromo-4-nitro-2,1,3-benzothiadiazole; 4,6-dichloro-5,7-dinitro-2,1,3-benzothiadiazole; 4,6-dibromo-5,7-dinitro-2,1,3-benzothiadiazole; 5-chloro-7-fluoro-4-nitro-2,1,3-benzothiadiazole; 5-anilino-7-bromo-4-nitro-2,1,3-benzothiadiazole; 5-amino-7-chloro-4-nitro-2,1,3-benzothiadiazole; 7-bromo-5-(diethylamino)-4-nitro-2,1,3-benzothiadiazole; 4-chloro-5-methyl-7-nitro-2,1,3-benzothiadiazole; 4-bromo-5-methyl-4-nitro-2,1,3-benzothiadiazole; 7-bromo-5-(bromomethyl)-7-nitro-2,1,3-benzothiadiazole; N-(7-chloro-4-nitro-2,1,3-benzothiadiazol-5-yl)acetamide; 4-chloro-7-nitro-2,1,3-benzoselenadiazole; 4-bromo-7-nitro-2,1,3-benzoselenadiazole; 4-methoxy-7-nitro-2,1,3-benzoselenadiazole; 4-ethoxy-7-nitro-2,1,3-benzoselenadiazole; 5,7-dibromo-4-nitro-2,1,3-benzoselenadiazole; 5,7-dichloro-4-nitro-2,1,3-benzoselenadiazole; 7-bromo-5-methyl-4-nitro-2,1,3-benzoselenadiazole and 7-bromo-5-(bromomethyl)-4-nitro-2,1,3-benzoselenadiazole.

3. Agent according to Claim 1, **characterized in that** the amine, aminonitrobenzene and phenol is chosen from ethanolamine, 1,4-diaminobenzene; 1,4-diamino-2-methylbenzene; 1,4-diamino-2-(2'-hydroxyethyl)benzene; 1,4-diamino-2-(1'-hydroxyethyl)benzene; 2,4-diamino-1-(2'-hydroxyethoxy)benzene; 2,4,5,6-tetraaminopyrimidine; hydroquinone; 5,6-diamino-2,4-dihydroxypyrimidine; 2,7-diaminofluorene; 1-amino-2-naphthol; 2,3-dihydro-3-methyl-2-benzothiazolone hydrazone; 2-aminophenol; 4,5-diamino-1-(2'-hydroxyethyl)-1H-pyrazole; 4,5-diamino-1-benzyl-1H-pyrazole; phenol; 4,5-diamino-1-((4'-methylbenzyl)-1H-pyrazole; 4,5-diamino-1H-pyrazole; 4,5-diamino-1-methyl-1H-pyrazole; 4,5-diamino-(1-isopropyl)-1H-pyrazole; N-(3-dimethylaminophenyl)urea; 2-amino-4-[(2'-hydroxyethyl)amino]-anisole; 1,3-diaminobenzene; 1,3-di(2,4-diaminophenoxy)propane; 4-methylphenol; 4-aminophenol; 4-amino-3-methylphenol; 5-amino-2-methylphenol; 3-aminophenol; 1,3-dihydroxybenzene; 5-hydroxyindole; 4-[(2'-hydroxyethyl)amino]-3-nitro-1-methylbenzene; 4-[(2'-hydroxyethyl)amino]-3-nitrophenol; 2-amino-6-chloro-4-nitrophenol; 2-chloro-6-ethylamino-4-nitrophenol; 1-amino-2-[(2'-hydroxyethyl)amino]-5-nitrobenzene, and 2,5-diaminonitrobenzene, such as, for example, 1-(2'-hydroxyethyl)amino-2-nitro-4-[di-(2'-hydroxyethyl)amino]benzene, 1-amino-4-[di-(2'-hydroxyethyl)amino]-2-nitrobenzene; 4-[di-(2'-hydroxyethyl)-amino]-1-[(2'-methoxyethyl)amino]-2-nitrobenzene, 4-[ethyl-(2'-hydroxyethyl)amino]-1-[(2'-hydroxyethyl)-amino]-2-nitrobenzene, 1-amino-5-chloro-4-[(2',3'-dihydroxypropyl)amino]-2-nitrobenzene and 2,5-diamino-6-nitropyridine.

4. Agent according to one of Claims 1 to 3, **characterized in that** it comprises the compounds of the formula (I), and the amines, aminonitrobenzenes and/or phenols, in the respective colour carrier mass (component (A1) or component (A2) respectively) in each case in a total amount of from 0.02 to 20 per cent by weight.

5. Agent according to one of Claims 1 to 4, **characterized in that** it comprises compounds of the formula (I), and the amines, aminonitrobenzenes and/or phenols, in the ready-to-use colourant (A) in each case in a total amount of from 0.01 to 10 per cent by weight.

6. Agent according to one of Claims 1 to 5, **characterized in that** it additionally comprises 0.02 to 20 per cent by weight of a physiologically acceptable, direct dye from the group of cationic and anionic dyes, disperse dyes, nitro dyes, azo dyes, quinone dyes and triphenylmethane dyes.

7. Agent according to one of Claims 1 to 6, **characterized in that** the ready-to-use colourant (A) has a pH of from 3 to 12.

8. Agent according to one of Claims 1 to 7, **characterized in that** it additionally comprises oxidation dye precursors.

9. Agent according to one of Claims 1 to 8, **characterized in that**, prior to use, it is mixed with an oxidizing agent.

10. Agent according to one of Claims 1 to 9, **characterized in that** it is a hair colourant.

11. Method of colouring hair in which the ready-to-use colourant (A) is prepared directly prior to use by mixing two components (A1) and (A2) - optionally with the addition of sodium acetate - and then applied to the hair and, after a contact time of from 5 to 60 minutes at a temperature of from 20 to 50°C, the hair is rinsed with water, optionally washed with a shampoo and then dried, **characterized in that** a colourant (A) according to one of Claims 1 to 10 obtainable by mixing two components (A1) and (A2) is used.

12. Multicomponent kit for colouring hair, consisting of an agent of component (A1) according to one of Claims 1 to 10 and an agent of component (A2) according to one of Claims 1 to 10, and optionally an agent for adjusting the pH.

13. Use of a combination of at least one compound of the formula (I), 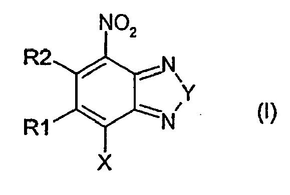 in which X is a halogen atom, a methoxy group or an ethoxy group; Y is an oxygen atom, a sulphur atom or a selenium atom; and R1 and R2 may be identical or different and, independently of one another, are hydrogen, a halogen atom, a (C₁-C₄)-alkyl group, a (C₁-C₄)-alkyl group substituted by a halogen atom, a (C₁-C₄)-alkoxy group, a nitro group, an acetamido group or an NR^{a}R^{b} group, where the radicals R^{a} and R^{b} may be identical or different and, independently of one another, are hydrogen, a (C₁-C₄)-alkyl group, an optionally substituted aromatic carbocycle or a (C₁-C₄)-alkanecarbonyl group, or R^{a} or R^{b}, together with the nitrogen atom, form a heterocyclic (C₃-C₆) radical; and at least one compound from the group consisting of amines, aminonitrobenzenes and phenols for colouring keratin fibres.

## Revendications

1. Composition (A) pour la teinture de fibres, qui est obtenue par mélange de deux composants (A1) et (A2) et est **caractérisée en ce que** le composant (A1) contient au moins un composé de formule (I), 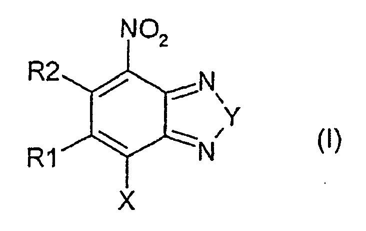 dans laquelle X représente un atome d'halogène, un groupe méthoxy ou un groupe éthoxy ; Y représente un atome d'oxygène, un atome de soufre ou un atome de sélénium ; et R1 et R2 peuvent être identiques ou différents et représentent, indépendamment l'un uns de l'autre, un atome d'hydrogène, un atome d'halogène, un groupe alkyle en C₁-C₄, un groupe alkyle en C₁-C₄ substitué par un atome d'halogène, un groupe alcoxy en C₁-C₄, un groupe nitro, un groupe acétamido ou un groupe NR^{a}R^{b}, les radicaux R^{a} et R^{b} pouvant être identiques ou différents et représentant, indépendamment l'un de l'autre, un atome d'hydrogène, un groupe alkyle en C₁-C₄, un cycle aromatique carbocyclique éventuellement substitué ou un groupe alcane(C₁-C₄)carbonyle, ou R^{a} et R^{b} pouvant former ensemble avec l'atome d'azote un radical hétérocyclique en C₃-C₆ ; et le composant (A2) contient au moins un composé choisi dans l'ensemble constitué par des amines, des aminonitrobenzènes et des phénols.

2. Composition selon la revendication 1, **caractérisée en ce que** le composé de formule (I) est choisi parmi des 4-nitro-2,1,3-benzoxadiazoles, des 4-nitro-2,1,3-benzothiadiazoles et des 4-nitro-2,1,3-benzosélénadiazoles, à savoir : le 4-chloro-7-nitro-2,1,3-benzoxadiazole, le 4-bromo-7-nitro-2,1,3-benzoxadiazole, le 4-fluoro-7-nitro-2,1,3-benzoxadiazole, le 4-méthoxy-7-nitro-2,1,3-benzoxadiazole, le 4-éthoxy-7-nitro-2,1,3-benzoxadiazole, le 4-chloro-5,7-dinitro-2,1,3-benzoxadiazole, le 5,7-dichloro-4-nitro-2,1,3-benzoxadiazole, le 5,7-dibromo-4-nitro-2,1,3-benzoxadiazole, le 7-chloro-4-nitro-5-diéthylamino-2,1,3-benzoxadiazole, le 5-amino-7-chloro-4-nitro-2,1,3-benzoxadiazole, le 7-chloro-4-nitro-5-(1-pipéridinyl)-2,1,3-benzoxadiazole, le 4-chloro-7-nitro-2,1,3-benzothiadiazole, le 4-bromo-7-nitro-2,1,3-benzothiadiazole, le 4-méthoxy-7-nitro-2,1,3-benzothiadiazole, le 4-éthoxy-7-nitro-2,1,3-benzoxathiadiazole, le 4-chloro-5,7-dinitro-2,1,3-benzothiadiazole, le 4-bromo-5,7-dinitro-2,1,3-benzothiadiazole, le 4,5-dichloro-7-nitro-2,1,3-benzothiadiazole, le 5,7-dichloro-4-nitro-2,1,3-benzothiadiazole, le 4,5-dibromo-7-nitro-2,1,3-benzothiadiazole, le 5,7-dibromo-4-nitro-2,1,3-benzothiadiazole, le 4,6-dichloro-5,7-dinitro-2,1,3-benzothiadiazole, le 4,6-dibromo-5,7-dinitro-2,1,3-benzothiadiazole, le 5-chloro-7-fluoro-4-nitro-2,1,3-benzothiadiazole, le 5-anilino-7-bromo-4-nitro-2,1,3-benzothiadiazole, le 5-amino-7-chloro-4-nitro-2,1,3-benzothiadiazole, le 7-bromo-5-(diéthylamino)-4-nitro-2,1,3-benzothiadiazole, le 4-chloro-5-méthyl-7-nitro-2,1,3-benzothiadiazole, le 4-bromo-5-méthyl-4-nitro-2,1,3-benzothiadiazole, le 7-bromo-5-(bromométhyl-7-nitro-2,1,3-benzothiadiazole, le N-(7-chloro-4-nitro-2,1,3-benzothiadiazol-5-yl)-acétamide, le 4-chloro-7-nitro-2,1,3-benzo-sélénadiazole, le 4-bromo-7-nitro-2,1,3-benzosélénadiazole, le 4-méthoxy-7-nitro-2,1,3-benzosélénadiazole, le 4-éthoxy-7-nitro-2,1,3-benzosélénadiazole, le 5,7-dibromo-4-nitro-2,1,3-benzosélénadiazole, le 5,7-Dichloro-4-nitro-2,1,3-benzosélénadiazole, le 7-bromo-5-méthyl-4-nitro-2,1,3-benzosélénadiazole et le 7-bromo-5-(bromométhyl)-4-nitro-2,1,3-benzo-sélénadiazole.

3. Composition selon la revendication 1, **caractérisée en ce que** l'amine, l'aminonitrobenzène et le phénol sont choisis parmi l'éthanolamine, le 1,4-diaminobenzène, le 1,4-diamino-2-méthyl-benzène, le 1,4-diamino-2-(2'-hydroxyéthyl)-benzène, le 1,4-diamino-2-(1'-hydroxyéthylbenzène, le 2,4-diamino-1-(2'-hydroxyéthoxy)-benzène, la 2,4,5,6-tétraamino-pyrimidine, l'hydroquinone, la 5,6-diamino-2,4-dihydroxy-pyrimidine, le 2,7-diaminofluorène, le 1-amino-2-naphtol, la 2,3-dihydro-3-méthyl-2-benzothiazolone-hydrazone, le 2-aminophénol, le 4,5-diamino-1-(2'-hydroxy-éthyl-1H-pyrazole, le 4,5-diamino-1-benzyl-1H-pyrazole, le phénol, le 4,5-diamino-1-((4'-méthylbenzyl)-1H-pyrazole, le 4,5-diamino-1H-pyrazole, le 4,5-diamino-1-méthyl-1H-pyrazole, le 4,5-diamino-(1-isopropyl)-1H-pyrazole, la N-(3-diméthylaminophényl)-urée, le 2-amino-4-[(2'-hydroxyéthyl)-amino]anisole, le 1,3-diaminobenzène, le 1,3-di(2,4-diamino-phénoxy)-propane, le 4-méthylphénol, le 4-aminophénol, le 4-amino-3-méthylphénol, le 5-amino-2-méthylphénol, le 3-aminophénol, le 1,3-dihydroxybenzène, le 5-hydroxyindole, le 4-[(2'-hydroxyéthyl)-amino]-3-nitro-1-méthyl-benzène, le 4-[(2'-hydroxyéthyl)amino]-3-nitro-phénol, le 2-amino-6-chloro-4-nitrophénol, le 2-chloro-6-éthylamino-4-nitrophénol, le 1-amino-2-[(2'-hydroxyéthy))amino]-5-nitrobenzène ainsi que des 2,5-diamino-nitrobenzènee, comme par exemple le 1-(2'-hydroxyéthyl)amino-2-nitro-4-[di-(2'-hydroxyéthyl)-amino]-benzène, le 1-amino-4-[di(2'-hydroxyéthyl)amino]-2-nitrobenzène, le 4-[di(2'-hydroxyéthyl)amino]-1-[(2'méthoxy-éthyl)-amino]-2-nitrobenzène, le 4-[éthyl-(2'-hydroxyéthyl)amino]-1-[(2'-hydroxyéthyl)-amino]-2-nitrobenzène, le 1-amino-5-chloro-4-[(2',3'-dihydroxypropyl)amino]-2-nitrobenzène et la 2,5-diamino-6-nitropyridine.

4. Composition selon l'une quelconque des revendications 1 à 3, **caractérisée en ce qu'**elle contient les composés de formule (I) ainsi que les amines, aminonitrobenzènes et/ou phénols dans la matière colorante [composant (A1) ou composant (A2)] chacun en une quantité totale de 0,02 à 20 % en poids.

5. Composition selon l'une quelconque des revendications 1 à 4, **caractérisée en ce qu'**elle contient les composés de formule (I) ainsi que les amines, aminonitrobenzènes et/ou phénols dans la composition de teinture (A) prête à l'emploi chacun en une quantité totale d'environ 0,01 à 10 % en poids.

6. Composition selon l'une quelconque des revendications 1 à 5, **caractérisée en ce qu'**elle contient en outre de 0,02 à 20% en poids d'un colorant direct physioloiquement sans inconvénient, choisi dans le groupe des colorants cationiques et des colorants anioniques, des colorants en dispersion, des colorants nitrés, des colorants azoïques, des colorants quinoniques et des colorants triphénylméthane.

7. Composition selon l'une quelconque des revendications 1 à 6, **caractérisée en ce que** la composition de teinture (A) prête à l'emploi présente un pH de 3 à 12.

8. Composition selon l'une quelconque des revendications 1 à 7, **caractérisée en ce qu'**elle contient en outre des précurseurs de colorants d'oxydation.

9. Composition selon l'une quelconque des revendications 1 à 8, **caractérisée en ce qu'**elle est mélangée avant l'emploi avec un oxydant.

10. Composition selon l'une quelconque des revendications 1 à 9, **caractérisée en ce qu'**elle est une composition de teinture pour cheveux.

11. Procédé pour la teinture des cheveux, dans lequel on prépare immédiatement avant l'emploi la composition de teinture (A) prête à l'emploi, par mélange de deux composants (A1) et (A2) - éventuellement avec addition d'acétate de sodium - et ensuite on l'applique sur les cheveux et, après un temps d'action de 5 à 60 minutes à une température de 20 à 50 °C, on rince les cheveux à l'eau, éventuellement on les lave avec un shampooing et ensuite on les sèche, **caractérisé en ce qu'**on utilise une composition de teinture (A) selon l'une quelconque des revendications 1 à 10, pouvant être obtenue par mélange de deux composants (A1) et (A2).

12. Nécessaire à plusieurs composants pour la teinture des cheveux, constitué d'un agent du composant (A1) selon l'une quelconque des revendications 1 à 10 et d'un agent du composant (A2) selon l'une quelconque des revendications 1 à 10, ainsi qu'éventuellement d'un agent pour l'ajustement du pH.

13. Utilisation d'une association d'au moins un composé de formule (I), 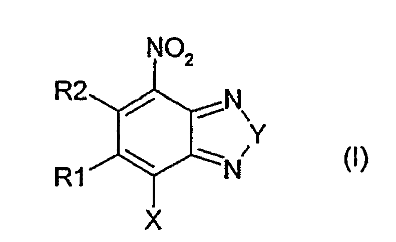 dans laquelle X représente un atome d'halogène, un groupe méthoxy ou un groupe éthoxy ; Y représente un atome d'oxygène, un atome de soufre ou un atome de sélénium ; et R1 et R2 peuvent être identiques ou différents et représentent, indépendamment l'un uns de l'autre, un atome d'hydrogène, un atome d'halogène, un groupe alkyle en C₁-C₄, un groupe alkyle en C₁-C₄ substitué par un atome d'halogène, un groupe alcoxy en C₁-C₄, un groupe nitro, un groupe acétamido ou un groupe NR^{a}R^{b}, les radicaux R^{a} et R^{b} pouvant être identiques ou différents et représentant, indépendamment l'un de l'autre, un atome d'hydrogène, un groupe alkyle en C₁-C₄, un cycle aromatique carbocyclique éventuellement substitué ou un groupe alcane(C₁-C₄)carbonyle, ou R^{a} et R^{b} pouvant former ensemble avec l'atome d'azote un radical hétérocyclique en C₃-C₆ ; et d'au moins un composé choisi dans l'ensemble constitué par des amines, des aminonitrobenzènes et des phénols,
pour la teinture de fibres de kératine.
